# EUROPÄISCHE PATENTSCHRIFT

(11) **EP 3 499 461 B1**
(45) Veröffentlichungstag und Bekanntmachung des Hinweises auf die Patenterteilung: **18.08.2021**
(21) Anmeldenummer: 17206883.5
(22) Anmeldetag: 13.12.2017
(51) Int. Cl.: G06T 7/70, G06T 7/00, A61B 6/12, A61B 90/00

(54) **DARSTELLUNG VON MARKERN IN DER MEDIZINISCHEN BILDGEBUNG**
REPRESENTATION OF MARKERS IN MEDICAL IMAGING
REPRÉSENTATION DE MARQUEURS EN IMAGERIE MÉDICALE

(43) Veröffentlichungstag der Anmeldung: 19.06.2019
(73) Patentinhaber: Siemens Healthcare GmbH, 91052 Erlangen (DE)
(72) Erfinder: Hörnig, Mathias, 91052 Erlangen (DE)

(56) Entgegenhaltungen:
- GB-A- 2 512 384
- US-A1- 2015 371 390
- US-A1- 2017 215 823

## Beschreibung

Die vorliegende Erfindung betrifft medizinische Bildgebungsverfahren, und insbesondere ein Verfahren zur Darstellung eines Markers in medizinischen Bildgebungsverfahren. Weiter werden ein entsprechendes bildgebendes System, ein Computerprogrammprodukt und ein elektronischer Datenträger bereitgestellt.

In der bildgebenden Diagnostik werden häufig physikalische Marker oder Clips (Metall) verwendet, um verdächtige Bereiche für die Untersuchung zu markieren oder Orientierungshilfen für Positionierungen zu liefern. Bekannte Beispiele sind für die Mammografie auf die Brust aufklebbare Metallmarker verdächtiger Bereiche oder Metallmarker für Rechts/Links in der Radiologie für verschiedenste Organe oder Körperregionen. Ein weiteres Beispiel sind Nippel-Clips, die insbesondere in den USA verwendet werden, um die Nippelposition zu markieren. Ein weiteres Beispiel sind chirurgische Eingriffe, die röntgenassistiert sind, wobei Bereiche markiert werden, die zu behandeln sind (z.B. rechts, links, ROI).

Ein Problem derartiger Marker, welche zumeist aus Metall, wie Blei oder chirurgischem Edelstahl sind, besteht darin, dass sie Artefakte in den Bildern verursachen. Bildverarbeitungsalgorithmen können diese hochkontrastigen Objekte nur unzureichend berücksichtigen, detektieren und von Bilddaten der Körperregionen trennen. Dementsprechend ist häufig eine Korrektursoftware zum Minimieren dieser Artefakte erforderlich.

Ein weiteres Problem dieser sogenannten Objektmarker besteht im Vertauschen der Seiten (z.B. rechts, links) durch den Anwender oder im inkorrekten Platzieren dieser Marker.

Ein zusätzliches Problem besteht darin, dass bestimmte Markierungen bisher nicht durch die Röntgenbildgebung erfasst und automatisiert in die diagnostischen Bilder übertragen werden können. Hierzu gehören beispielhaft nichtmetallische Marker oder Markerstifte, welche vermehrt Einsatz in der Bildgebung finden, um zusätzliche Information an einer Untersuchungsperson anzubringen. Auch Kunststoffmarker können mit bestimmten bildgebenden Verfahren nicht, oder nicht deutlich, dargestellt werden. Damit fehlt ein wichtiger Link, um für die Diagnostik, Therapie aber auch Kontrolle, Dokumentation und Qualität und Qualitätssicherung von Untersuchungen verbessern zu können.

Die GB 2512384 A beschreibt einen Marker, der verwendet wird, um ein MR System und ein Röntgensystem zu kalibrieren. Es besteht Bedarf nach einem verbesserten Verfahren zur Darstellung eines Markers in der medizinische Bildgebung, welches die obengenannten Nachteile nicht aufweist, wobei insbesondere die Darstellung des Markers ohne Artefakte möglich ist.

Aus der Druckschrift US 2017/0215823 A1 ist ein Abbildungssystem und ein zugehöriges Verfahren bekannt, bei dem ein Marker-Detektionssystem einen oder mehrere Marker detektiert, die räumlich in Verbindung mit einem Bereich angeordnet sind. Wenn das Marker-Detektionssystem detektiert, dass sich der Bereich innerhalb eines Sichtfeldes (FoV) des Abbildungssystems oder zumindest innerhalb eines vordefinierten Abstands davon befindet, wird ein Steuersignal in Bezug auf den Bereich an ein Bilderfassungssystem des Abbildungssystems ausgegeben.
Diese Aufgabe wird mit den Merkmalen der unabhängigen Ansprüche gelöst. In den abhängigen Ansprüchen sind weitere Ausführungsformen der Erfindung beschrieben.

Gemäß einem ersten Aspekt der Erfindung wird ein Verfahren zur Darstellung eines Markers, welcher an einem Untersuchungsobjekt in einem bildgebenden System angebracht ist, bereitgestellt. In einem ersten Schritt wird die Position des Markers durch ein erstes Messverfahren bestimmt. In einem weiteren Schritt wird ein Bild des Untersuchungsobjekts basierend auf einem zweiten Messverfahren bereitgestellt. In einem zusätzlichen Schritt wird die Position eines graphischen Objekts, welches den Marker im Bild repräsentiert, im Bild basierend auf dem ersten Messverfahren bestimmt. In einem weiteren Schritt wird das graphische Objekt an der bestimmten Position in dem Bild dargestellt, wobei das graphische Objekt basierend auf dem zweiten Messverfahren nicht in dem Bild des Untersuchungsobjektes enthalten ist.

Die Vorteile des erfindungsgemäßen Verfahrens bestehen darin, dass Fehler, z.B. ein Vertauschen von Seiten, etwa links/rechts, vermieden werden können. Zudem wird durch zwei Messverfahren, beispielsweise zwei bildgebende Verfahren statt einem, die Genauigkeiten der Positionierung und Ausrichtung von Markierungen in den Untersuchungsbildern, sowie die Bildqualität verbessert, da beispielhaft nichtmetallische Marker, wie etwa Lotionen und Pflaster, in der Röntgenuntersuchung verwendet werden können, die von der Röntgenstrahlung nicht erfasst werden können, aber dennoch als graphische Objekte in Röntgenbilder projiziert und damit im Bild sichtbar sind. Insbesondere können durch Verwendung von nichtmetallischen Markern Artefakte in Röntgenbildern vermieden werden. Dadurch werden insgesamt die Sicherheit und die Qualität von Untersuchungen und Behandlungen verbessert.

Weiterhin kann ein derartiges Verfahren als Qualitätssicherheits- und Dokumentationsverfahren eingesetzt werden, wobei Marker, welche beispielsweise während diagnostischer oder chirurgischer Maßnahmen an einem Patienten angebracht wurden, in einer Patientenakte dokumentiert und dadurch während einer Kontrolle oder einer Nachuntersuchung verwendet werden können. Ein zusätzlicher Vorteil besteht darin, dass keine gesonderte Hardware erforderlich ist, da bildgebende medizinische Systeme zunehmend über mindestens zwei unterschiedliche bildgebende Verfahren, insbesondere auch über optische Kameras, verfügen, beispielsweise zur Ortung von Detektoren oder ähnlichen Komponenten des bildgebenden Systems. Dadurch wird eine weitere Anwendung und Nutzung derartiger integrierter Kameras für die klinische Prozedur und deren Verbesserung bereitgestellt.

Zusätzlich oder statt der räumlichen Position kann eine räumliche Orientierung oder eine räumliche Abmessung des Markers bestimmt werden, wobei in entsprechender Weise eine Orientierung oder eine Abmessung des graphischen Objekts in dem Bild bestimmt werden kann, mit welcher das graphische Objekt in dem Bild dargestellt werden kann.

Das erste und das zweite Messverfahren können unterschiedliche Messverfahren sein, welche auf unterschiedlichen physikalischen Messprinzipien beruhen.

Das erste und das zweite Messverfahren können im Wesentlichen gleichzeitig durchgeführt werden.

Das erste Messverfahren kann ein Ausführen eines Sprachbefehls von einer Bedienungsperson des bildgebenden Systems mittels Spracherkennung umfassen.

Das Darstellen des graphischen Objekts kann ein Erzeugen eines separaten Bildes des graphischen Objekts und ein Überlagern des Bildes des graphischen Objekts mit dem Bild des Untersuchungsobjekts umfassen.

Das erste Messverfahren kann eine Bilderfassung mittels einer optischen Kamera, und das zweite Messverfahren ein bildgebendes Röntgenverfahren umfassen.

Gemäß einem weiteren Aspekt wird ein bildgebendes System zur Darstellung eines Markers, welcher an einem Untersuchungsobjekt angebracht ist, bereitgestellt, wobei das bildgebende System eine Steuereinheit und eine Speichereinheit umfasst, wobei die Speichereinheit von der Steuereinheit ausführbare Steuerinformationen speichert, und wobei das bildgebende System ausgebildet ist, bei der Ausführung der Steuerinformationen in der Steuereinheit ein Verfahren mit den folgenden Schritten auszuführen. In einem ersten Schritt wird die Position des Markers durch ein erstes Messverfahren bestimmt. In einem weiteren Schritt wird ein Bild des Untersuchungsobjekts basierend auf einem zweiten Messverfahren bereitgestellt. In einem zusätzlichen Schritt wird die Position eines graphischen Objekts, welches den Marker im Bild repräsentiert, im Bild basierend auf dem ersten Messverfahren bestimmt. In einem weiteren Schritt wird das graphische Objekt an der bestimmten Position in dem Bild dargestellt, wobei das graphische Objekt basierend auf dem zweiten Messverfahren nicht in dem Bild des Untersuchungsobjektes enthalten ist.

Das bildgebende System kann ausgebildet sein, bei der Ausführung der Steuerinformationen in der Steuereinheit ein Verfahren entsprechend der weiteren unter dem ersten Aspekt der Erfindung beschriebenen Merkmale auszuführen.

Gemäß einem weiteren Aspekt der Erfindung wird ein Computerprogrammprodukt bereitgestellt, welches ein Programm umfasst, das direkt in einen Speicher einer Steuereinheit eines bildgebenden Systems ladbar ist, mit Programm-Mitteln, um die Schritte des Verfahrens entsprechend den unter dem ersten Aspekt der Erfindung beschriebenen Merkmale auszuführen, wenn das Programm in der Steuereinheit des bildgebenden Systems ausgeführt wird.

Gemäß einem weiteren Aspekt der Erfindung wird ein elektronisch lesbarer Datenträger mit darauf gespeicherten elektronisch lesbaren Steuerinformationen bereitgestellt, welche derart ausgestaltet sind, dass sie bei Verwendung des Datenträgers in einer Steuereinheit eines bildgebenden Systems das Verfahren entsprechend den unter dem ersten Aspekt der Erfindung beschriebenen Merkmalen durchführen.

Für ein derartiges bildgebendes System, Computerprogrammprodukt und elektronisch lesbaren Datenträger können technische Effekte erzielt werden, die vergleichbar sind mit den technischen Effekten, die für das Verfahren gemäß dem ersten Aspekt obenstehend beschrieben wurden.

Die oben dargelegten Merkmale und Merkmale, die nachfolgend beschrieben werden, können nicht nur in den entsprechenden explizit dargelegten Kombinationen verwendet werden, sondern auch in weiteren Kombinationen oder isoliert, ohne den Schutzumfang der vorliegenden Erfindung zu verlassen.

### Kurze Beschreibung der Figuren

Die Erfindung wird nachfolgend unter Bezugnahme auf die beiliegenden Zeichnungen näher erläutert.
Figur 1 zeigt schematisch ein bildgebendes System, mit welchem ein Verfahren zur Darstellung eines Markers erfindungsgemäß durchgeführt werden kann.
Figur 2 zeigt eine schematische Zeichnung eines graphischen Objektes, welches in einem Bild einer Untersuchungsperson dargestellt wird.
Figur 3 zeigt ein Flussdiagramm mit Schritten zur Durchführung eines Verfahrens zur Darstellung eines Markers gemäß einem Ausführungsbeispiel der Erfindung.

### Detaillierte Beschreibung

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Im Zusammenhang der vorliegenden Erfindung umfassen bildgebende Verfahren, welche mittels bildgebender Systeme 10 durchgeführt werden, als Oberbegriff verschiedene apparative Untersuchungsmethoden, welche zwei- oder dreidimensionale Bilddaten von Organen und Strukturen einer Untersuchungsperson 12, oder allgemeiner eines Untersuchungsobjekts 12, liefern.

Bildgebenden Verfahren können nach der Art ihrer Erfassung von Messdaten auf verschiedenen physikalischen Prinzipien, bzw. Effekten basieren. So können Messdaten zur Bilderzeugung mit Hilfe von Röntgenstrahlung (z.B. Röntgenaufnahmen, Computertomographie), Radionukliden (z.B. Szintigraphie), Ultraschall, Kernspinresonanz (z. B. Magnetresonanztomographie), Infrarotstrahlung (z. B. diagnostische Thermographie), oder sichtbarem Licht (z. B. Endoskopie, optische Tomographie, Videorasterstereographie) erfasst werden.

Die Auswahl eines bildgebenden Systems 10, bzw. der bildgebenden Messmethode beruht oft auf den Anforderungen, die der medizinischen Diagnostik gestellt werden. So werden beispielsweise Knochen in Röntgenaufnahmen gut dargestellt, die Szintigraphie kann unter anderem die Aktivitätsverteilung in der Schilddrüse darstellen. Die meisten Verfahren liefern statische Aufnahmen. Bewegte Bilder auch während Operationen können beispielsweise Ultraschall, Durchleuchtung, Endoskopie, und auch die Magnetresonanztomographie erzeugen.

Im Zusammenhang der vorliegenden Erfindung bezeichnen Marker 11 physikalische Marker, also physikalische Objekte oder Substanzen, welche verwendet werden, um zusätzliche Informationen an einem Untersuchungsobjekt 12, oder Patienten, anzubringen. Beispielsweise umfassen derartige physikalische Marker Objekte aus Metall, wie Blei oder chirurgischem Edelstahl, oder Plastik, sie können ebenfalls Pflastermarkierungen, oder Markierungen mittels eines Stiftes, Markers, oder einer Lotion auf der Untersuchungsperson, beispielhaft auf der Haut eines Patienten, umfassen. Derartige Zusatzinformationen können beispielsweise in der Diagnostik oder Chirurgie erforderlich sein. Insbesondere kann der Marker ausgewählt sein aus einer Gruppe, bestehend aus einer farbliche Markierung, einer optischen Markierung, einer Infrarotmarkierung, einer Ultraviolettmarkierung, einer Lasermarkierung, einem spezifisches Muster oder einer spezifische Form des Untersuchungsobjekts.

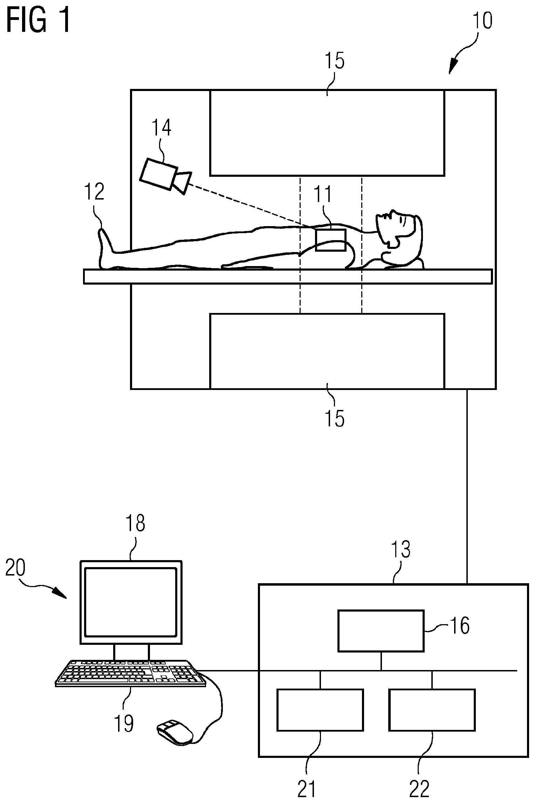

Figur 1 zeigt schematisch ein bildgebendes System 10, mit der ein Verfahren zur Darstellung eines Markers 11 erfindungsgemäß durchgeführt werden kann.

Eine Untersuchungsperson 12, oder allgemeiner ein Untersuchungsobjekt 12, ist in das bildgebende System 10 gefahren. Das bildgebende System weist ein erstes Messsystem 14 zum Durchführung eines ersten Messverfahrens, und ein zweites Messsystem 15 zum Durchführen eines zweiten Messverfahrens auf. In der der Figur 1 das erste Messsystem 14 eine Bilderfassung mittels einer optischen Kamera, welche im sichtbaren oder im Infrarotbereich arbeiten kann, und das zweite Messsystem 15 ist beispielsweise ein Röntgensystem. Jedoch kann eine beliebige andere Kombination von Messverfahren zur Erfassung eines Markers und zur Erzeugung eines Bildes des Untersuchungsobjektes vorgenommen werden, insbesondere können zwei beliebige bildgebende Verfahren miteinander kombiniert werden. Beispielsweise kann das erste Messsystem 14 ein optisches oder Infrarot-Kamerasystem sein, und das zweite Messsystem 15 eine Röntgenanlage oder eine MRT-Anlage. Derartige bildgebende Systeme sind dem Fachmann bekannt, sodass auf eine detaillierte Erläuterung hiervon verzichtet wird.

Das bildgebende System 10 weist weiterhin eine Steuereinheit 13 auf, die zur Steuerung des bildgebenden Systems verwendet wird. Die zentrale Steuereinheit 13, welche derart ausgebildet ist, dass sie das unten beschriebene Verfahren zur Darstellung eines Markers 11 durchführt, weist eine erste Steuerung 21 für das Messsystem 14 und eine zweite Steuerung 22 für das Messsystem 15 auf. In einer Speichereinheit 16 können beispielsweise die für die Aufnahme der Untersuchungsbilder notwendigen Steuerbefehle abgespeichert werden, insbesondere alle Programme, die zum Betrieb des bildgebenden Systems 10 notwendig sind. In einer Recheneinheit 20 können Bilder berechnet werden, die auf eine Anzeige 18 angezeigt werden können, wobei eine Bedienperson über eine Eingabeeinheit 19 das bildgebende System 10 bedienen kann. Die Speichereinheit 16 kann Steuerbefehle und Programmmodule aufweisen, die bei Ausführung in der Recheneinheit 20 das erfindungsgemäße Verfahren durchführen. Insbesondere speichert die Speichereinheit 16 dazu von der Steuereinheit 13 ausführbare Steuerinformationen.

Erfindungsgemäß ist das bildgebende System 10 der Figur 1 derart ausgebildet, dass es bei der Ausführung der Steuerinformationen in der Steuereinheit 13 einen an der Untersuchungsperson 12 befestigen Marker 11 vermisst und in einem Bild 1 der Untersuchungsperson 12 als graphisches Objekt 2 darstellt. Die Grundidee des erfindungsgemäßen Verfahrens, welches im Folgenden im Detail beschrieben wird, ist dabei die Darstellung eines graphischen Objektes in einem Bild 1 eines Untersuchungsobjektes 12, wobei die Messung der bildgebenden Daten und die Erfassung des Markers 11 in unterschiedlichen Messverfahren durchgeführt werden.

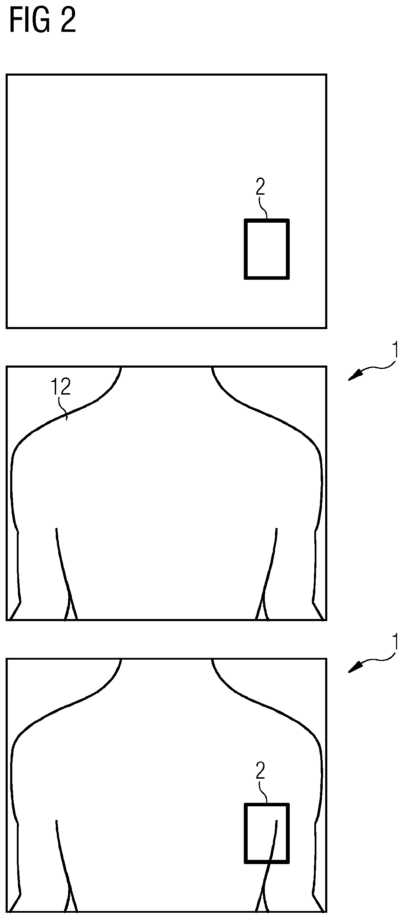

Figur 2 zeigt eine schematische Zeichnung eines graphischen Objektes, welches in einem Bild 1 einer Untersuchungsperson dargestellt wird.

In dem oberen Teil der Figur 2 ist die Position eines graphischen Objektes 2 in einem Bild gezeigt, welches basierend auf einem ersten Messvorgang bestimmt wurde. In dem mittleren Teil der Figur 2 ist ein Bild 1 einer Untersuchungsperson 12 gezeigt, welches durch ein zweites Messverfahren aufgenommen wurde. In dem Bild 1 ist ein Marker 11, welcher an der Untersuchungsperson angebracht ist, nicht abgebildet. In dem unteren Teil der Figur 2 ist das graphische Objekt 2 in dem Bild 1 der Untersuchungsperson dargestellt, wobei die Position des graphischen Objektes 2 der Position des Markers 11 an der Untersuchungsperson 12 entspricht.

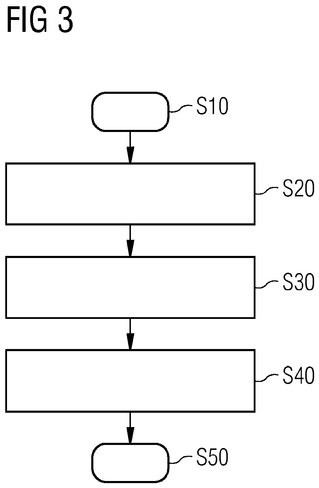

Figur 3 zeigt ein Flussdiagramm mit Schritten zur Durchführung eines Verfahrens zur Darstellung eines Markers 11 gemäß einem Ausführungsbeispiel der Erfindung.

Das Verfahren beginnt in Schritt S10. In Schritt S20, wird ein Marker 11, welcher an einer Untersuchungsperson 12, oder allgemeiner an einem Untersuchungsobjekt 12, in einem bildgebenden System 10 angebracht ist, durch ein erstes Messverfahren erfasst. Insbesondere umfassen die Messdaten des ersten Messvorgangs Daten, welche auf dem Marker 11 basieren, und mit welchen Charakteristiken des Markers 11, wie etwa eine räumliche Position, eine räumliche Orientierung, und eine räumliche Abmessung des Markers 11 bestimmt werden können. Diese Charakteristiken können relativ zu dem Untersuchungsobjekt 11, oder in einem anderen Ausführungsbeispiel auch bezüglich eines Koordinatensystems des bildgebenden Systems 10 bestimmt werden. Dabei können nur eine Charakteristik, eine beliebige Kombination der Charakteristiken, oder alle der genannten Charakteristiken bestimmt, und in Folge mittels des graphischen Objekts 2 dargestellt werden.

In einem Ausführungsbeispiel umfasst das erste Messverfahren zusätzlich ein Erfassen eines Sprachbefehls von einer Bedienungsperson des bildgebenden Systems. Der erfasste Sprachbefehl kann mittels Spracherkennung interpretiert und ausgeführt werden, so dass mündlich oder akustisch gegebene Informationen der Bedienungsperson in das erste Messverfahren und auch in die Auswahl und Erzeugung eines graphischen Objektes eingeht.

In Schritt S30 wird ein Bild 1 des Untersuchungsobjektes 12 bereitgestellt. Das Bild 1 des Untersuchungsobjekts 12 basiert dabei auf Messdaten eines bildgebenden zweiten Messverfahrens. In einem erfindungsgemäßen Ausführungsbeispiel umfassen die Messdaten des zweiten Messverfahrens im Wesentlichen keine Daten des Markers 11, d.h. keine Daten, welche auf dem physikalischen Marker 11 beruhen, obwohl sich der Marker 11 im Untersuchungsbereich des zweiten Messverfahrens befindet. Dies kann durch geeignete Auswahl des Markers mit Bezug zu dem zweiten bildgebenden Verfahren ermöglicht werden. In einem anderen Ausführungsbeispiel können die Messdaten des zweiten Messvorgangs zwar Daten des physikalischen Markers 11 enthalten, jedoch sind diese Daten nicht ausreichend, so dass der Marker 11 nicht in dem Bild 1 abgebildet werden kann, wobei im Wesentlichen keine Artefakte durch den Marker 11 im Bild 1 hervorgerufen werden.

In einem Ausführungsbeispiel beruhen das erste und das zweite bildgebende Verfahren auf unterschiedlichen physikalischen Messprinzipien, oder in anderen Worten auf unterschiedlichen physikalischen Grundlagen der Messung, also auf unterschiedlichen physikalischen Effekten. Unterschiedliche Messprinzipien sind dem Fachmann beispielsweise aus der Norm DIN 1319 bekannt. Beispielhaft beruhen das erste Messverfahren auf einer Messung mittels einer oder mehrerer Kameras 14 im optisch sichtbaren Lichtbereich oder im Infrarotbereich, und das zweite Messverfahren auf einer Messung mittels eines Röntgensystems 15. Das erste Messverfahren und das zweite Messverfahren können bildgebende Verfahren sein.

In einem anderen Ausführungsbeispiel beruhen das erste und das zweite Messverfahren auf dem gleichen physikalischen Messprinzip, aber unterschiedlichen Messvorgängen, oder auf im Wesentlichen unterschiedlichen Messdaten. In einem weiteren Ausführungsbeispiel kann das erste Messverfahren ein Subset der Messdaten des zweiten Messverfahrens umfassen, beispielsweise Daten einer Röntgenstrahlung mit einer spezifischen Energie oder Frequenz.

In einem Ausführungsbeispiel werden die Messungen für das erste und das zweite bildgebende Verfahren gleichzeitig durchgeführt.

In Schritt S40 wird ein graphisches Objekt 2 in dem Bild 1 des Untersuchungsobjektes 12 dargestellt. Das graphische Objekt 2 beruht in einem Ausführungsbeispiel nicht auf dem zweiten Messverfahren, d.h. nicht auf den Messdaten des zweiten Messverfahrens, und in einem weiteren Ausführungsbeispiel nur auf den Messdaten des ersten Messverfahrens. In diesem Zusammenhang handelt es sich in einem Ausführungsbeispiel bei dem graphischen Objekt 2 um eine virtuelle Markierung, oder einen virtuellen Marker, welche derart bestimmt werden, dass sie der Position, Orientierung, oder Abmessung des Markers entsprechen, also im Bild 1 für einen Betrachter erkenntlich darstellen. Insbesondere kann das graphische Objekt 2 derart in dem Bild 1 dargestellt werden, dass die Position des graphischen Objektes zu dem abgebildeten Untersuchungsobjekt 12 in dem Bild 1, der Position des Markers 11 zum Untersuchungsobjekt in dem bildgebenden System 10 entspricht. Entsprechendes gilt für die räumliche Orientierung und die räumliche Abmessung des graphischen Objektes 2, sofern diese durch das erste Messverfahren erfasst wurden. Dadurch wird der Marker 11 und das Bild 1 des Untersuchungsobjektes 11 durch unterschiedliche Messverfahren erfasst und für den Benutzer des bildgebenden Systems erkenntlich im Bild 1 dargestellt. Das Verfahren endet in Schritt S50.

Das Darstellen des graphischen Objekts 2 kann in einem Ausführungsbeispiel ein Einbringen als Annotation, Einblenden, Projizieren, oder Überlagern des graphischen Objekts 2 in bzw. auf das Bild 1 des Untersuchungsobjektes 12 umfassen. Weiter können die Messdaten des ersten Messverfahrens mit den Messdaten des zweiten Messverfahrens registriert werden, oder in anderen Worten, miteinander verknüpft, oder miteinander in Beziehung gebracht werden.

In einem Ausführungsbeispiel kann das graphische Objekt 2 zusammen mit dem Bild 1 des Untersuchungsobjekts 12 abgespeichert werden. In einem anderen Ausführungsbeispiel wird ein separates Bild des graphischen Objekts 2 erzeugt, und mit dem Bild 1 des Untersuchungsobjekts 12 überlagert. Das Bild des graphischen Objekts 2 kann separat oder überlagert mit dem Bild 1 des Untersuchungsobjektes gespeichert werden.

In einem Ausführungsbeispiel erfolgt die Erfassung metallischer wie nichtmetallischer physikalischer Marker 11 über eines oder eine beliebige Kombination der folgenden Verfahren, wie zum Beispiel eine optische oder farbliche Erkennung etwa durch optische Kameras, Röntgenmessungen, spektrale Röntgenmessungen, welche energiespezifisch durchgeführt werden, Infraroterkennung oder Ultravioletterkennung mittels einer Kamera, Laser, Muster- und Formerkennung mit Hilfe einer optischen Kamera oder mit Hilfe von Röntgenaufnahmen.

In einem Ausführungsbeispiel basiert die Erfassung des Markers 11 oder die Darstellung des graphischen Objekts 2 auf einem gewählten Organprogramm des bildgebenden Systems 10. Beispielsweise wird durch die Berücksichtigung des gewählten Organprogramms eine Vorauswahl von möglichen graphischen Objekten 2, oder ein Ausschluss von nicht zutreffenden graphischen Objekten 2, vorgenommen.

In einem Ausführungsbeispiel basiert die Erfassung des Markers 11 oder die Darstellung des graphischen Objekts 2 auf einer vergleichenden Objekterkennung, welche auf optischen Messungen oder mit Hilfe von Röntgenaufnahmen durchgeführt werden kann. Beispielsweise wird in dem ersten Messvorgang eine handschriftliche Eingabe einer Bedienperson empfangen, welche mittels Objekterkennung identifiziert und einem vorbestimmten graphischen Objekt 2 zugeordnet wird.

In einem Ausführungsbeispiel ist das bildgebende System 10 ein medizinisches Bildgebungssystem, ein diagnostisches System oder ein therapeutisches System. Weiter kann das bildgebende System derart ausgebildet sein, dass es neben beispielsweise der Röntgenbilderfassung eines der obengenannten Verfahren, insbesondere eine Bildanalyse optischer Bilder, ausführt. Zusätzlich verfügt das System über ein Registrierungsverfahren dieser Erfassungsverfahren mit den Röntgenbildern. In einem Ausführungsbeispiel kann der virtuelle Marker, oder in anderen Worten das graphische Objekt 2, über einen lernenden Algorithmus trainiert werden.

In einem weiteren Ausführungsbeispiel kann das Darstellen des graphischen Objekts 2 basierend auf einem physikalischen Marker 11, insbesondere anhand einer Markierung auf der Haut durch beispielsweise einen Stift, einen Marker Pen oder einen Luminescent Pen, trainiert werden, oder zusätzlich autonom selbstlernend verbessert werden. Zum Erfassen eines virtuellen Markers in klinischen Bildern, insbesondere bei Röntgenbildern wird in einem Ausführungsbeispiel eine Creme oder Lotion auf dem betroffenen Bereich als physikalischer Marker aufgebracht.

In einem Ausführungsbeispiel kann der Anwender dem System den verwendeten physikalischen Marker 11 oder Markertyp mitteilen, woraufhin das System dann automatisch spezifisch für den gewählten Typ eine Analyse durchführt, beispielsweise berücksichtigt das System bei der Verwendung einer Lotion als Marker eine Infrarotkamera bei der Analyse.

In einem weiteren Ausführungsbeispiel kann der virtuelle Marker als separates Bildmaterial, beispielsweise als komplette oder teilkomplette optische Bilder, ausgeführt werden und ohne zusätzliche Verfahrensschritte mit den Röntgenbildern überlagert, und abgespeichert werden. In einem Ausführungsbeispiel kann der virtuelle Marker in 2D oder 3D erfasst und dokumentiert werden.

Zusammenfassend wird ein Verfahren zur graphischen Darstellung eines Markers, welcher an einem Untersuchungsobjekt in einem bildgebenden System angebracht ist, bereitgestellt. Dabei wird die Position des Markers durch ein erstes Messverfahren bestimmt. Basierend auf einem zweiten Messverfahren wird ein Bild des Untersuchungsobjekts bereitgestellt, in welchem die Position eines graphischen Objekts, welches den Marker im Bild repräsentiert, basierend auf dem ersten Messverfahren dargestellt wird. In dem erfindungsgemäßen Verfahren, wird durch zwei Messverfahren die Genauigkeiten der Positionierung und Ausrichtung eines graphischen Objekts, welches den Marker in dem Bild des Untersuchungsobjektes repräsentiert, verbessert.

### Bezugszeichenliste

- 1: Bild
- 2: Figur

- 10: bildgebendes System
- 11: Marker
- 12: Untersuchungsobjekt
- 13: Steuereinheit
- 14: erstes Messsystem
- 15: zweites Messsystem
- 16: Speichereinheit

- 18: Anzeige
- 19: Eingabeeinheit
- 20: Recheneinheit
- 21: erste Steuerung
- 22: zweite Steuerung

## Patentansprüche

1. Verfahren zur Darstellung eines Markers (11), welcher an einem Untersuchungsobjekt (12) in einem bildgebenden System (10) angebracht ist, umfassend die folgenden Schritte:
- Bestimmen der Position des Markers (11) durch ein erstes Messverfahren;
- Bereitstellen eines Bildes (1) des Untersuchungsobjekts (12) basierend auf einem zweiten Messverfahren;
- Bestimmen der Position eines graphischen Objekts (2), welches den Marker (11) in dem Bild (1) repräsentiert, basierend auf dem ersten Messverfahren;
**dadurch gekennzeichnet, dass** das Verfahren ferner den folgenden Schritt umfasst:
- Darstellen des graphischen Objekts (2) an der bestimmten Position in dem Bild (1), wobei das graphische Objekt (2) basierend auf dem zweiten Messverfahren nicht in dem Bild (1) des Untersuchungsobjektes (12) enthalten ist und wobei Messdaten des zweiten Messverfahrens keine Daten des Markers (11) umfassen, obwohl sich der Marker (11) im Untersuchungsbereich des zweiten Messverfahrens befindet.

2. Verfahren nach Anspruch 1, wobei zusätzlich eine Orientierung oder eine Abmessung des Markers (11) bestimmt wird, wobei eine Orientierung oder eine Abmessung des graphischen Objekts (2) in dem Bild (1) bestimmt wird, und wobei das das graphische Objekt (2) mit der bestimmten Orientierung oder Abmessung in dem Bild (1) dargestellt wird.

3. Verfahren nach Anspruch 1, wobei das erste und das zweite Messverfahren unterschiedliche Messverfahren sind, welche auf unterschiedlichen physikalischen Messprinzipien beruhen.

4. Verfahren nach einem der vorhergehenden Ansprüche, wobei das erste und das zweite Messverfahren im Wesentlichen gleichzeitig durchgeführt werden.

5. Verfahren nach einem der vorhergehenden Ansprüche, wobei das erste Messverfahren ein Ausführen eines Sprachbefehls von einer Bedienungsperson des bildgebenden Systems (10) mittels Spracherkennung umfasst.

6. Verfahren nach einem der vorhergehenden Ansprüche, wobei das Darstellen des graphischen Objekts (2) ein Erzeugen eines separaten Bildes des graphischen Objekts (2) und ein Überlagern des Bildes des graphischen Objekts (2) mit dem Bild (1) des Untersuchungsobjekts (12) umfasst.

7. Verfahren nach einem der vorhergehenden Ansprüche, wobei das erste Messverfahren eine Bilderfassung mittels einer optischen Kamera, und das zweite Messverfahren ein bildgebendes Röntgenverfahren umfasst.

8. Bildgebendes System zur Darstellung eines Markers (11), welcher an einem Untersuchungsobjekt (12) angebracht ist, umfassend eine Steuereinheit (13) und eine Speichereinheit (16), wobei die Speichereinheit (16) von der Steuereinheit (13) ausführbare Steuerinformationen speichert, und wobei das bildgebende System (10) ausgebildet ist, bei der Ausführung der Steuerinformationen in der Steuereinheit (13) das Verfahren mit den folgenden Schritten auszuführen:
- Bestimmen der Position des Markers (11) durch ein erstes Messverfahren;
- Bereitstellen eines Bildes (1) des Untersuchungsobjekts (12) basierend auf einem zweiten Messverfahren;
- Bestimmen der Position eines graphischen Objekts (2), welches den Marker (11) in dem Bild (1) repräsentiert, basierend auf dem ersten Messverfahren;
**dadurch gekennzeichnet, dass** das Verfahren ferner den folgenden Schritt umfasst:
- Darstellen des graphischen Objekts (2) an der bestimmten Position in dem Bild, wobei das graphische Objekt (2) basierend auf dem zweiten Messverfahren nicht in dem Bild (1) des Untersuchungsobjektes (12) enthalten ist und wobei Messdaten des zweiten Messverfahrens keine Daten des Markers (11) umfassen, obwohl sich der Marker (11) im Untersuchungsbereich des zweiten Messverfahrens befindet.

9. Bildgebendes System umfassend eine Steuereinheit (13) und eine Speichereinheit (16), wobei die Speichereinheit (16) von der Steuereinheit (13) ausführbare Steuerinformationen speichert, und wobei das bildgebende System (10) ausgebildet ist, bei der Ausführung der Steuerinformationen in der Steuereinheit (13) das Verfahren eines der Ansprüche 2 bis 7 auszuführen.

10. Computerprogrammprodukt umfassend ein Programm, welches direkt in einen Speicher einer Steuereinheit (13) eines bildgebenden Systems (10) ladbar ist, mit Programm-Mitteln, um die Schritte des Verfahrens nach einem der Ansprüche 1 bis 7 auszuführen, wenn das Programm in der Steuereinheit (13) des bildgebenden Systems (10) ausgeführt wird.

11. Elektronisch lesbarer Datenträger mit darauf gespeicherten elektronisch lesbaren Steuerinformationen, welche derart ausgestaltet sind, dass sie bei Verwendung des Datenträgers in einer Steuereinheit (13) eines bildgebenden Systems (10) das Verfahren nach einem der Ansprüche 1 bis 7 durchführen.

## Claims

1. Method for depicting a marker (11) which is applied to an examination object (12) in an imaging system (10), comprising the following steps:
- ascertaining the position of the marker (11) by means of a first measuring method;
- providing an image (1) of the examination object (12) on the basis of a second measuring method;
- ascertaining the position of a graphical object (2), which represents the marker (11) in the image (1), on the basis of the first measuring method;
**characterised in that** the method further comprises the following step:
- depicting the graphical object (2) at the ascertained position in the image (1), wherein the graphical object (2) is not contained in the image (1) of the examination object (12) on the basis of the second measuring method and wherein measured data from the second measuring method comprises no data of the marker (11), although the marker (11) is situated in the field of view of the second measuring method.

2. Method according to claim 1, wherein an orientation or a dimension of the marker (11) is additionally ascertained, wherein an orientation or a dimension of the graphical object (2) in the image (1) is ascertained, and wherein the graphical object (2) is depicted with the ascertained orientation or dimension in the image (1).

3. Method according to claim 1, wherein the first and the second measuring methods are different measuring methods which are based on different physical measuring principles.

4. Method according to one of the preceding claims, wherein the first and the second measuring methods are performed at essentially the same time.

5. Method according to one of the preceding claims, wherein the first measuring method includes execution of a voice command of an operator of the imaging system (10) by means of voice recognition.

6. Method according to one of the preceding claims, wherein the depiction of the graphical object (2) comprises generating a separate image of the graphical object (2) and superimposing the image of the graphical object (2) on the image (1) of the examination object (12).

7. Method according to one of the preceding claims, wherein the first measuring method includes image capture by means of an optical camera and the second measuring method comprises an imaging X-ray method.

8. Imaging system for depicting a marker (11) which is applied to an examination object (12), comprising a control unit (13) and a memory unit (16), wherein the memory unit (16) stores control information which can be executed by the control unit (13), and wherein the imaging system (10) is designed to execute the method comprising the following steps when the control information is executed in the control unit (13) :
- ascertaining the position of the marker (11) by means of a first measuring method;
- providing an image (1) of the examination object (12) on the basis of a second measuring method;
- ascertaining the position of a graphical object (2), which represents the marker (11) in the image (1), on the basis of the first measuring method;
**characterised in that** the method further comprises the following step:
- depicting the graphical object (2) at the ascertained position in the image, wherein the graphical object (2) is not contained in the image (1) of the examination object (12) on the basis of the second measuring method and wherein measured data from the second measuring method comprises no data of the marker (11), although the marker (11) is situated in the field of view of the second measuring method.

9. Imaging system comprising a control unit (13) and a memory unit (16), wherein the memory unit (16) stores control information that can be executed by the control unit (13), and wherein the imaging system (10) is designed to execute the method according to one of claims 2 to 7 when the control information is executed in the control unit (13).

10. Computer program product comprising a program which can be loaded directly into a memory of a control unit (13) of an imaging system (10), having program means for executing the steps of the method according to one of claims 1 to 7 when the program is executed in the control unit (13) of the imaging system (10).

11. Electronically readable data medium on which is stored electronically readable control information that is configured in such a way as to perform the method according to one of claims 1 to 7 when the data medium is used in a control unit (13) of an imaging system (10).

## Revendications

1. Procédé de représentation d'un repère (11), qui est mis sur un objet (12) à examiner dans un système (10) d'imagerie, comprenant les stades suivants :
- on détermine la position du repère (11) par un premier procédé de mesure ;
- on prépare une image (1) de l'objet (12) à examiner sur la base d'un deuxième procédé de mesure ;
- on détermine la position d'un objet (2) graphique, qui représente le repère (11) dans l'image (1), sur la base du premier procédé de mesure ;
**caractérisé en ce que** le procédé comprend en outre le stade suivant :
- on représente l'objet (2) graphique en la position déterminée dans l'image (1), l'objet (2) graphique n'étant, sur la base du deuxième procédé de mesure, pas contenu dans l'image (1) de l'objet (12) à examiner et des données de mesure du deuxième procédé de mesure ne comprenant pas de données du repère (11), bien que le repère (11) se trouve dans le domaine d'examen du deuxième procédé de mesure.

2. Procédé suivant la revendication 1, dans lequel on détermine en outre une orientation ou une dimension du repère (11), une orientation ou une dimension de l'objet (2) graphique étant déterminée dans l'image (1) et l'objet (2) graphique étant représenté dans l'image (1) en ayant l'orientation ou la dimension déterminée.

3. Procédé suivant la revendication 1, dans lequel le premier et le deuxième procédés de mesure sont des procédés de mesure différents, qui reposent sur des principes physiques de mesure différents.

4. Procédé suivant l'une des revendications précédentes, dans lequel on effectue sensiblement en même temps le premier et le deuxième procédé de mesure.

5. Procédé suivant l'une des revendications précédentes, dans lequel le premier procédé de mesure comprend l'exécution d'une instruction vocale d'une personne de service du système (10) d'imagerie au moyen d'une reconnaissance vocale.

6. Procédé suivant l'une des revendications précédentes, dans lequel la représentation de l'objet (2) graphique comprend une production d'une image distincte de l'objet (2) graphique et une superposition de l'image de l'objet (2) graphique à l'image (1) de l'objet (12) à examiner.

7. Procédé suivant l'une des revendications précédentes, dans lequel le premier procédé de mesure comprend une prise d'image au moyen d'un appareil photographique optique et le deuxième procédé de mesure un procédé d'imagerie à rayons X.

8. Système d'imagerie pour la représentation d'un repère (11), qui est mis sur un objet (12) à examiner, comprenant une unité (13) de commande et une unité (16) de mise en mémoire, dans lequel l'unité (16) de mise en mémoire met en mémoire des informations de commande pouvant être exécutées par l'unité (13) de commande et dans lequel le système (10) d'imagerie est constitué pour, à l'exécution des informations de commande dans l'unité (13) de commande, effectuer le procédé ayant les stades suivants :
- on détermine la position du repère (11) par un premier procédé de mesure ;
- on prépare une image (1) de l'objet (12) à examiner sur la base d'un deuxième procédé de mesure ;
- on détermine la position d'un objet (2) graphique, qui représente le repère (11) dans l'image (1), sur la base du premier procédé de mesure ;
**caractérisé en ce que** le procédé comprend en outre le stade suivant :
- on représente l'objet (2) graphique en la position déterminée dans l'image, l'objet (2) graphique n'étant, sur la base du deuxième procédé de mesure, pas contenu dans l'image (1) de l'objet (12) à examiner et des données de mesure du deuxième procédé de mesure ne comprenant pas de données du repère (11), bien que le repère (11) se trouve dans le domaine d'examen du deuxième procédé de mesure.

9. Système d'imagerie comprenant une unité (13) de commande et une unité (16) de mise en mémoire, l'unité (16) de mise en mémoire mettant en mémoire des informations de commande pouvant être exécutées par l'unité (13) de commande, et dans lequel le système (10) d'imagerie est constitué pour, lors de l'exécution des informations de commande dans l'unité (13) de commande, effectuer le procédé suivant l'une des revendications 2 à 7.

10. Produit de programme d'ordinateur comprenant un programme, qui peut être chargé directement dans une mémoire d'une unité (13) de commande d'un système (10) d'imagerie et qui a des moyens de programme pour effectuer les stades du procédé suivant l'une des revendications 1 à 7, lorsque le programme est réalisé dans l'unité (13) de commande du système (10) d'imagerie.

11. Support de données déchiffrable électroniquement, sur lequel sont mises en mémoire des informations de commande déchiffrables électroniquement, qui sont conformées de manière à ce qu'elles effectuent, lors de l'utilisation du support de données dans une unité (13) de commande d'un système (10) d'imagerie, le procédé suivant l'une des revendications 1 à 7.
